(19) Europäisches Patentamt
European Patent Office
Office européen des brevets

(11) Veröffentlichungsnummer: **0 039 905**
**B1**

# (12) EUROPÄISCHE PATENTSCHRIFT

(45) Veröffentlichungstag der Patentschrift: **02.11.83**

(51) Int. Cl.³: **C 07 D 277/68**

(21) Anmeldenummer: **81103452.9**

(22) Anmeldetag: **07.05.81**

## (54) Verfahren zur Herstellung von 2-Chlorbenzothiazol.

(30) Priorität: **12.05.80 DE 3018088**

(43) Veröffentlichungstag der Anmeldung: **18.11.81 Patentblatt 81/46**

(45) Bekanntmachung des Hinweises auf die Patenterteilung: **02.11.83 Patentblatt 83/44**

(84) Benannte Vertragsstaaten: **AT BE CH DE FR GB IT LI NL SE**

(56) Entgegenhaltungen:
**DE - A - 1 670 706**
**DE - A - 2 213 865**
**DE - A - 2 451 632**

(73) Patentinhaber: **CASSELLA Aktiengesellschaft, Hanauer Landstrasse 526, D-6000 Frankfurt am Main 61 (DE)**

(72) Erfinder: **Kühlein, Klaus, Dr., Fasanenstrasse 41, D-6233 Kelkheim/Taunus (DE)**
Erfinder: **Kussmaul, Ulrich, Dr., Ulmenweg 1, D-6369 Niederdorfelden (DE)**

(74) Vertreter: **Urbach, Hans-Georg, Dr. et al, Hanauer Landstrasse 526, D-6000 Frankfurt am Main 61 (DE)**

**EP 0 039 905 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 2-Chlorbenzothiazol

Die Erfindung betrifft ein Verfahren zur Herstellung eines 2-Chlorbenzothiazols der Formel I

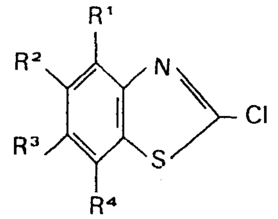

(I)

worin $R^1$, $R^2$, $R^3$, $R^4$ unabhängig voneinander jeweils ein Wasserstoff- oder Halogenatom, vorzugsweise ein Wasserstoff- oder Chloratom, bedeuten.

2-Chlorbenzothiazole der Formel I sind wichtige Zwischenprodukte, insbesondere für die Herstellung von Farbstoffen und Pflanzenschutzmitteln, und können nach verschiedenen bekannten Verfahren hergestellt werden, die jedoch für die technische Herstellung mit verschiedenen Mängeln behaftet sind. So ist es z.B. bekannt, dass 2-Chlorbenzothiazole ausgehend von 2-Mercaptobenzothiazolen durch Chlorierung mit verschiedenen Chlorierungsmitteln, wie z.B. Chlor (DE-AS Nr. 1670453), Thionylchlorid (Houben-Weyl, „Methoden der org. Chemie", Bd. V/3 [1962], S. 871), Dischwefeldichlorid oder Schwefeldichlorid (US-PS Nr. 2043948), Sulfurylchlorid (Modica, Barni und Magrassi, „Ann. Chimica", 53 [1963], Nr. 6, S. 733-740), Phosgen (DE-AS Nr. 1164413) hergestellt werden können. Bei der Herstellung der als Ausgangsprodukte benötigten 2-Mercaptobenzothiazole erfordert die Verwendung von Schwefelkohlenstoff jedoch besondere sicherheitstechnische Massnahmen, wodurch die Wirtschaftlichkeit des Gesamtverfahrens beeinträchtigt wird.

Aus der DE-A Nr. 2212865 ist ein Verfahren zur Herstellung von in 2,5-Stellung speziell substituierten 4-Chlorthiazolen bekannt, bei dem Isocyaniddichloride, die an den der Isocyaniddichloridgruppe benachbarten α- und β-Kohlenstoffatomen jeweils mindestens ein Chlor besitzen, mit Schwefel umgesetzt werden. Es war zu erwarten, dass ein Ringschluss nur bei den speziell substituierten Ausgangsprodukten möglich ist.

Aus der DE-A1 Nr. 2451632 ist ein Verfahren zur Herstellung von Trichlorthiazol bekannt, bei dem 1,2-Dichloräthylisocyaniddichlorid bei erhöhter Temperatur in Gegenwart katalytischer Mengen eines Friedel-Crafts-Katalysators mit Schwefelchloriden umgesetzt wird.

2-Chlorbenzothiazole können auch durch Sandmeyer-Reaktion aus 2-Aminobenzothiazolen hergestellt werden (vgl. Modica, Barni und Magrassi *loc. cit.* und DE-A1 Nr. 2360202, GB-PS Nr. 966496). Die Abspaltung der Aminogruppe durch Diazotierung ist jedoch wenig wirtschaftlich. 2-Chlorbenzothiazole können auch direkt durch Erhitzen von 2,6-Dichlorphenylisothiocyanaten hergestellt werden, wozu jedoch in jedem Falle hohe Temperaturen von 330 bis 380° C erforderlich sind („Angew. Chem.", 82 [1970], 79 und DE-A Nr. 1670706). Bei dem gegebenen Stand der Technik war demnach nicht zu erwarten, dass sich 2-Chlorbenzothiazole direkt in einfacher und wirtschaftlicher Weise bereits bei Temperaturen unter 300° C aus 2-Chlorphenylisocyaniddichloriden in Ausbeuten von 89% der Theorie und mehr herstellen lassen.

Das erfindungsgemässe Verfahren zur Herstellung von 2-Chlorbenzothiazol der allgemeinen Formel I ist dadurch gekennzeichnet, dass ein 2-Chlorphenylisocyanidichlorid der Formel II

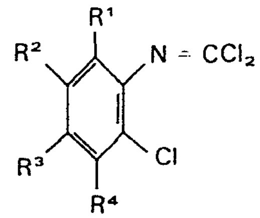

(II)

in der $R^1$, $R^2$, $R^3$ und $R^4$ die oben angegebene Bedeutung besitzen, bei Temperaturen von 150 bis 400° C mit Schwefel im Molverhältnis von mindestens 1 : 1,5 umgesetzt und das bei der Umsetzung erhaltene Reaktionsgemisch anschliessend bei Temperaturen von −20 bis 300° C mit einem Chlorierungsmittel versetzt wird.

Entsprechend der folgenden Reaktionsgleichung:

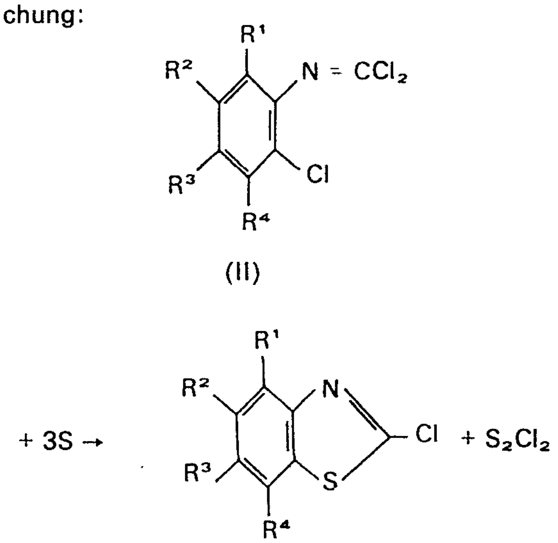


(II)

(I)

werden für die Umsetzung pro Mol Ausgangsverbindung der Formel II stöchiometrisch 3 mol Schwefel benötigt. Es können aber auch andere Molverhältnisse von Ausgangsverbindung zu Schwefel eingesetzt werden und dann die jeweils überschüssige Komponente gegebenenfalls in den Reaktionskreislauf zurückgeführt werden. Im allgemeinen beträgt das Molverhältnis zwischen dem 2-Chlorphenylisocyaniddichlorid der Formel II und Schwefel 1 : (1,5 bis 10), insbesondere 1 : (2,4 bis 5). Besonders gute Ergebnisse erhält man bei Molverhältnissen von 1 : (2,7 bis 4).

Zur Durchführung des erfindungsgemässen Verfahrens wird das 2-Chlorphenylisocyaniddichlorid der Formel II mit dem Schwefel vermischt und zweckmässigerweise unter Rühren auf Temperaturen von 150 bis 400° C, vorzugsweise 220 bis 300° C, erhitzt. Die Erhitzung des Gemisches kann im offenen oder geschlossenen Reaktionsgefäss erfolgen. Falls gewünscht, kann die Reaktion auch unter der Atmosphäre eines Inertgases, z.B. von Stickstoff oder eines Edelgases, wie z.B. Argon, durchgeführt werden. Die Reaktionszeit beträgt im allgemeinen 1 bis 24 h je nach Ausgangsprodukt und der zur Anwendung kommenden Temperatur. In vielen Fällen ist die Reaktion bereits nach 10 bis 12 h beendet.

Da bei der Durchführung der Reaktion im geschlossenen Gefäss, d.h. unter Überdruck, gegebenenfalls zusätzliche Kernhalogenierungen eintreten können, wird das erfindungsgemässe Verfahren vorzugsweise bei Normaldruck, d.h. vorzugsweise im offenen Gefäss, durchgeführt.

Bei der Durchführung der Reaktion im offenen Gefäss werden die gebildeten Schwefelchloride zweckmässigerweise laufend abdestilliert. Die Menge der abdestillierten Schwefelchloride zeigt den Fortgang der Reaktion an.

Nach Beendigung der Umsetzung wird das bei der Reaktion gebildete schwefelhaltige, noch nicht aufgearbeitete Reaktionsgemisch, das unerwünschte Nebenprodukte, z.B. solche mit Disulfidbrücken und acyclische, schwefelhaltige Produkte, enthält, mit einem Chlorierungsmittel oder einem Gemisch verschiedener Chlorierungsmittel versetzt. Pro Mol Ausgangsverbindung der Formel II werden etwa 0,2 bis 4 mol, vorzugsweise 0,5 bis 1,5 mol, Chlorierungsmittel zugefügt. Durch die Chlorierung werden die unerwünschten Nebenprodukte in Endprodukte der Formel I und Ausgangsprodukte der Formel II überführt. Als Chlorerungsmittel wird zweckmässigerweise elementares Chlor verwendet, geeignet sind aber auch andere chlorierend wirkende Substanzen, wie z.B. Sulfurylchlorid, Thionylchlorid oder Phosphorpentachlorid. Chlor wird bis zur Sättigung in das Reaktionsgemisch eingeleitet. Die Chlorierung kann zweckmässigerweise unter Rühren bei Temperaturen von −20 bis 300° C, vorzugsweise bei 0 bis 100° C, erfolgen. Gegebenenfalls ist bei der Chlorierung die Temperatur so niedrig zu wählen, dass keine Kernhalogenierung in den Verbindungen der Formel I oder II erfolgt.

Zur Isolierung des Endprodukts der Formel I werden zunächst die Schwefelchloride abgetrennt, was z.B. durch fraktionierte Destillation erfolgen kann, und dann wird das Endprodukt der Formel I von dem Ausgangsprodukt der Formel II in geeigneter Weise, z.B. durch Destillation, Sublimation oder Kristallisation getrennt. Das Ausgangsprodukt der Formel II kann dann gegebenenfalls erneut in den Reaktionskreislauf zurückgeführt werden.

Das erfindungsgemässe Verfahren kann diskontinuierlich oder kontinuierlich durchgeführt werden. Die Anwesenheit eines Lösungsmittels ist nicht erforderlich. Gewünschtenfalls kann das erfindungsgemässe Verfahren aber auch in einem geeigneten inerten Lösungsmittel durchgeführt werden, das einen entsprechend hohen Siedepunkt besitzt. Geeignete Lösungsmittel sind z.B. übliche Lösungsmittel für die Halogenierung, wie hochsiedende Halogenkohlenwasserstoffe, wie z.B. Chlornaphthaline.

Die benötigten Ausgangsverbindungen der Formel II sind bekannt und nach verschiedenen Methoden in einfacher Weise zugänglich (DE-AS Nr. 1094737, E. Kühle *et al.*, „Angew. Chem.", *79*, 663 [1967]). Als Beispiele seien genannt: 2-Chlorphenylisocyaniddichlorid, 2,3-Dichlorphenylisocyaniddichlorid, 2,4-Dichlorphenylisocyaniddichlorid, 2,5-Dichlorphenylisocyaniddichlorid, 2,4,5-Trichlorphenylisocyaniddichlorid, 2,3,4,5-Tetrachlorphenylisocyaniddichlorid, Pentachlorphenylisocyaniddichlorid, 2-Chlor-4-fluorphenylisocyaniddichlorid, 4-Brom-2-chlorphenylisocyaniddichlorid, 2-Chlor-4,5-dibromphenylisocyaniddichlorid, 2-Chlor-4-jodphenylisocyaniddichlorid.

Das erfindungsgemässe Verfahren liefert die Verbindungen der Formel I in Ausbeuten von 89% der Theorie und mehr.

*Beispiel 1*

60,7 g (250 mmol) 2,4-Dichlorphenylisocyaniddichlorid werden mit 24,0 g (750 mVal) Schwefel (Molverhältnis 1:3) versetzt und dann unter Rühren 10 h auf 255 bis 270° C erhitzt. Dabei destillieren 24,4 g Schwefelchlorid vom Siedepunkt 125 bis 135° C ab. Man lässt das Reaktionsgemisch auf 85° C abkühlen und leitet unter Rühren bei dieser Temperatur so lange Chlor ein, wie es aufgenommen wird (Gewichtszunahme des Reaktionsgemisches: 12,2 g, Dauer ca. 2 h). Dann wird erneut Schwefelchlorid abdestilliert und der Rückstand im Wasserstrahlvakuum fraktioniert destilliert. Es werden

12,8 g 2,4-Dichlorphenylisocyaniddichlorid vom Siedepunkt 126 bis 127° C bei 13,3 mbar, und

36,1 g 2,6-Dichlorbenzothiazol vom Siedepunkt 140 bis 141° C bei 13,3 mbar, und vom Schmelzpunkt 97 bis 98° C erhalten, entsprechend einer Ausbeute von 89%, bezogen auf umgesetztes Ausgangsprodukt.

Die Herstellung weiterer, in der nachfolgenden Tabelle zusammengefasster 2-Chlorbenzothiazole erfolgt analog der in Beispiel 1 beschriebenen Arbeitsweise mit ähnlich guten Ausbeuten.

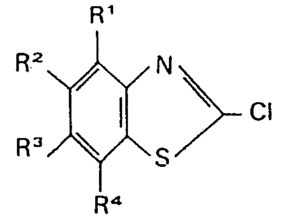

| Beispiel | $R^1$ | $R^2$ | $R^3$ | $R^4$ |
|---|---|---|---|---|
| 2 | H | H | H | H |
| 3 | Cl | H | H | H |
| 4 | H | Cl | H | H |

| Beispiel | $R^1$ | $R^2$ | $R^3$ | $R^4$ |
|---|---|---|---|---|
| 5 | H | H | H | Cl |
| 6 | H | Cl | Cl | H |
| 7 | H | Cl | Cl | Cl |
| 8 | Cl | Cl | Cl | Cl |
| 9 | H | H | F | H |
| 10 | H | H | Br | H |
| 11 | H | Br | Br | H |
| 12 | H | H | J | H |

**Patentansprüche**

1. Verfahren zur Herstellung von 2-Chlorbenzothiazol der Formel I

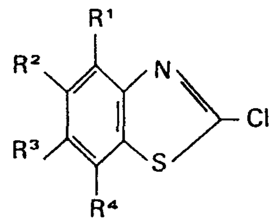

(I)

wobei $R^1$, $R^2$, $R^3$ und $R^4$ unabhängig voneinander für ein Wasserstoffatom oder Halogenatom stehen, dadurch gekennzeichnet, dass ein 2-Chlorphenylisocyaniddichlorid der Formel II

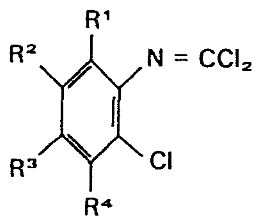

(II)

in der $R^1$, $R^2$, $R^3$ und $R^4$ die oben angegebene Bedeutung besitzen, bei Temperaturen von 150 bis 400° C mit Schwefel im Molverhältnis von 1 : (1,5 bis 10) umgesetzt und das bei der Umsetzung erhaltene Reaktionsgemisch anschliessend bei Temperaturen von −20 bis 300° C mit einem Chlorierungsmittel versetzt wird.

2. Verfahren nach Anspruch 1, dadurch gekennzeichnet, dass die Umsetzung mit Schwefel bei Temperaturen von 220 bis 300° C durchgeführt wird.

3. Verfahren nach den Ansprüchen 1 und/oder 2, dadurch gekennzeichnet, dass das Molverhältnis zwischen dem Phenylisocyaniddichlorid der Formel II und dem Schwefel 1 : (2,4 bis 5) beträgt.

4. Verfahren nach einem oder mehreren der Ansprüche 1 bis 3, dadurch gekennzeichnet, dass das Molverhältnis zwischen dem Phenylisocyaniddichlorid der Formel II und dem Schwefel 1 : (2,7 bis 4) beträgt.

5. Verfahren nach einem oder mehreren der Ansprüche 1 bis 4, dadurch gekennzeichnet, dass das bei der Umsetzung erhaltene Reaktionsgemisch bei Temperaturen von 0 bis 100° C mit einem Chlorierungsmittel versetzt wird.

6. Verfahren nach einem oder mehreren der Ansprüche 1 bis 5, dadurch gekennzeichnet, dass als Chlorierungsmittel elementares Chlor verwendet wird.

7. Verfahren nach Anspruch 6, dadurch gekennzeichnet, dass das Chlor bis zur Sättigung in das Reaktionsgemisch eingeleitet wird.

8. Verfahren nach einem oder mehreren der Ansprüche 1 bis 7, dadurch gekennzeichnet, dass aus dem Reaktionsgemisch Phenylisocyaniddichlorid der Formel II zurückgewonnen und von neuem mit Schwefel umgesetzt wird.

9. Verfahren nach einem oder mehreren der Ansprüche 1 bis 8, dadurch gekennzeichnet, dass ein Phenylisocyaniddichlorid der Formel II eingesetzt wird, bei dem $R^1$, $R^2$, $R^3$, $R^4$ unabhängig voneinander ein Wasserstoff- oder Chloratom bedeuten.

**Claims**

1. Process for the manufacture of 2-chlorobenzothiazole of the formula I

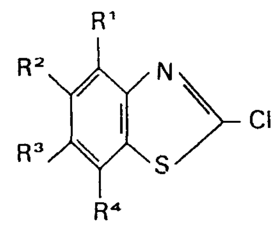

(I)

wherein $R^1$, $R^2$, $R^3$ and $R^4$ independently of one another represent a hydrogen atom or halogen atom, characterised in that a 2-chlorophenyl isocyanide dichloride of the formula II

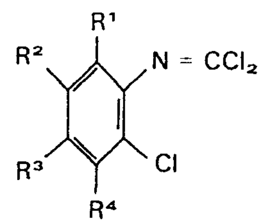

(II)

in which $R^1$, $R^2$, $R^3$ and $R^4$ have the above-mentioned meaning, is reacted with sulphur in a molar ratio of at least 1 : (1.5 to 10) at temperatures from 150 to 400° C and a chlorinating agent is subsequently added, at temperatures from −20 to 300° C, to the reaction mixture obtained in the reaction.

2. Process according to claim 1, characterised in that the reaction with sulphur is carried out at temperatures from 220 to 300° C.

3. Process according to claims 1 and/or 2, characterised in that the molar ratio between the phenyl isocyanide dichloride of the formula II and the sulphur is 1 : (2.4 to 5).

4. Process according to one or several of claims 1 to 3, characterised in that the molar ratio between the phenyl isocyanide dichloride of the formula II and the sulphur is 1 : (2.7 to 4).

5. Process according to one or several of claims 1 to 4, characterised in that a chlorinating agent is added at temperatures from 0 to 100° C, to the reaction mixture obtained in the reaction.

6. Process according to one or several of claims 1 to 5, characterised in that elementary chlorine is used as the chlorinating agent.

7. Process according to claim 6, characterised in that the chlorine is passed into the reaction mixture until saturation is reached.

8. Process according to one or several of claims 1 to 7, characterised in that phenyl isocyanide dichloride of the formula II is recovered from the reaction mixture and is reacted again with sulphur.

9. Process according to one or several of claims 1 to 8, characterised in that a phenyl isocyanide dichloride of the formula II in which $R^1$, $R^2$, $R^3$ and $R^4$ independently of one another denote a hydrogen or chlorine atom, is employed.

## Revendications

1. Procédé de préparation de chloro-2 benzothiazole répondant à la formule (I):

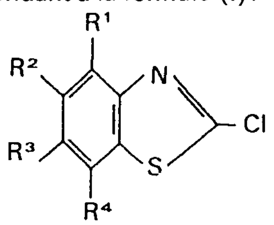
(I)

dans laquelle $R^1$, $R^2$, $R^3$ et $R^4$ représentent chacun, indépendamment les uns des autres, un atome d'hydrogène ou un atome d'halogène, procédé caractérisé en ce qu'on fait réagir un dichlorure de (chloro-2 phényl)carbonimidoyle répondant à la formule (II):

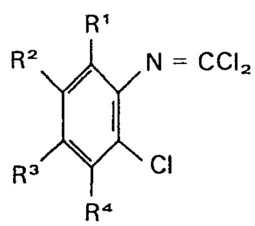
(II)

dans laquelle $R^1$, $R^2$, $R^3$ et $R^4$ ont les significations précédemment données, à des températures de 150 à 400° C, avec du soufre, dans un rapport molaire compris entre 1:1,5 et 1:10, puis, au mélange réactionnel obtenu lors de la réaction, on ajoute un agent de chloration, à des températures de −20 à 300° C.

2. Procédé selon la revendication 1, caractérisé en ce qu'on effectue la réaction avec le soufre à des températures de 220 à 300° C.

3. Procédé selon l'une quelconque des revendications 1 et 2, caractérisé en ce que le rapport molaire entre le dichlorure de phénylcarbonimidoyle de formule (II) et le soufre est compris entre 1:2,4 et 1:5.

4. Procédé selon l'une quelconque des revendications 1 à 3, caractérisé en ce que le rapport molaire entre le dichlorure de phénylcarbonimidoyle de formule (II) et le soufre est compris entre 1:2,7 et 1:4.

5. Procédé selon l'une quelconque des revendications 1 à 4, caractérisé en ce que l'addition de l'agent de chloration au mélange obtenu lors de la réaction est effectuée à des températures de 0 à 100° C.

6. Procédé selon l'une quelconque des revendications 1 à 5, caractérisé en ce qu'on utilise du chlore élémentaire comme agent de chloration.

7. Procédé selon la revendication 6, caractérisé en ce que le chlore est introduit dans le mélange réactionnel jusqu'à la saturation.

8. Procédé selon l'une quelconque des revendications 1 à 7, caractérisé en ce qu'on récupère, à partir du mélange réactionnel, le dichlorure de phénylcarbonimidoyle de formule (II) et qu'on le fait réagir à nouveau avec du soufre.

9. Procédé selon l'une quelconque des revendications 1 à 8, caractérisé en ce qu'on utilise un dichlorure de phénylcarbonimidoyle de formule (II) dans lequel $R^1$; $R^2$, $R^3$ et $R^4$ représentent chacun, indépendamment les uns des autres, un atome d'hydrogène ou un atome de chlore.